# EUROPEAN PATENT SPECIFICATION

(11) **EP 1 347 773 B1**
(45) Date of publication and mention of the grant of the patent: **14.04.2010**
(21) Application number: 02707393.1
(22) Date of filing: 03.01.2002
(51) Int. Cl.: A61K 38/19, A61P 35/00, A61K 31/555

(54) **COMPOSITIONS AND METHODS FOR ENHANCING CYTOKINE ACTIVITY AND TREATING HYPOTENSION ASSOCIATED WITH THE ADMINISTRATION OF CYTOKINES**
ZUSAMMENSETZUNGEN UND VERFAHREN ZUR ERHÖHUNG DER ZYTOKIN-AKTIVITÄT UND ZUR BEHANDLUNG DER HYPOTENSION ASSOZIIERT MIT DER VERABREICHUNG VON ZYTOKINEN
COMPOSITIONS ET METHODES PERMETTANT D'AMELIORER L'ACTIVITE D'UNE CYTOKINE ET DE TRAITER L'HYPOTENSION ASSOCIEE A L'ADMINISTRATION DE CYTOKINES

(30) Priority: 05.01.2001 US 260120 P
(43) Date of publication of application: 01.10.2003
(73) Proprietor: MetaPhore Pharmaceuticals, Inc., St. Louis, MO 63114 (US)
(72) Inventor: SALVEMINI, Daniela, Chesterfield, MO 63017 (US)
(74) Representative: Smaggasgale, Gillian Helen
(86) International application number: PCT/US2002/000036
(87) International publication number: WO 2002/053142

(56) References cited:
- WO-A-89/11277
- WO-A-97/33588
- K.I. TODA E.A.: "UVB/PUVA-induced suppression of human natural killer activity is reduced by superoxide dismutase and /or interleukin 2 in vitro" JOURNAL OF INVESTIGATIVE DERMATOLOGY, vol. 86, no. 5, 1986, pages 519-522, XP008009802
- W.E.SAMLOWSKI E.A.: "The SOD mimetic M40403 blocks IL-2 induced hypotension and increases anticancer responses" PROCEEDINGS OF THE AMERICAN ASSOCIATION FOR CANCER RESEARCH ANNUAL MEETING, vol. 42, 2001, page 502 XP008009749
- HENKE S.L.: 'Superoxide dismutase mimics as future therapeutics' EXPERT OPINION ON THERAPEUTIC PATENTS vol. 9, no. 2, February 1999, pages 169 - 180, XP000905307
- SALVEMINI: 'M40403 SUPEROXIDE DISMUTASE MIMETIC' DRUGS OF THE FUTURE vol. 25, no. 10, 2000, PROUS SCIENCE, ES, pages 1027 - 1033, XP009052060
- MACARTHUR H. ET AL: 'INACTIVATION OF CATECHOLAMINES NY SUPEROXIDE GIVES NEW INSIGHTS ON THE PATHOGENESIS OF SEPTIC SHOCK' PROCEEDINGS OF THE NATIONAL ACADEMY OF SCIENCES OF USA vol. 97, no. 17, 15 August 2000, WASHINGTON, USA, pages 9753 - 9758, XP002188830

## Description

### Field of Invention

This invention relates to compositions for use in methods for enhancing the immune activity of a interleukin-2 (IL-2) by co-administering the IL-2 with therapeutic amounts of catalysts for the dismutation of superoxide. This invention also relates to compositions for use in methods of preventing and treating hypotension in a mammal resulting from the administration of IL-2 by the administration of therapeutic amounts of catalysts for the dismutation of superoxide in combination with a IL-2. Also disclosed herein are methods of enhancing cancer treatments in a subject by the administration of therapeutic amounts of catalysts for the dismutation of superoxide in combination with a IL-2. Also provided are pharmaceutical compositions comprising IL-2 and catalysts for the dismutation of superoxide alone or in combination with catecholamine pressor agents for use in these methods.

### Background of the Invention

In the immune system, the three major types of lymphocytes are B cells, T cells, and natural killer (NK) cells. B-cells are derived from bone marrow and comprise about 10% of the lymphocytes found circulating in blood. When stimulated by a specific antigen, each B-cell differentiates into a plasma cell that secretes antibodies of a single specificity. T-cells mature in the thymus and make up about 80% of circulating lymphocytes. T-cells have specific antigen receptors resembling antibody molecules on their surfaces and react to antigen stimulation by secreting immunomediator molecules or cytokines and toxic molecules. Cytotoxic T-cells secrete toxic molecules that kill infected cells and any foreign particles that they may contain, such as microorganisms. NK cells make up about 10% of the lymphocyte population, are not antigen specific and recognize and kill cells infected with microbes.

Monocytes and macrophages are large scavenger cells that ingest foreign particles and present antigens to the T-cells which trigger specific immune responses. When an antigen is introduced, it is initially ingested by macrophages and other antigen presenting cells. After digestion, short segments thereof are presented on their cell surfaces. Only a few of all circulating T-cells have receptors that specifically bind to the antigen and this binding stimulates the T-cells to secrete cytokines.

Cytokines are small proteins secreted primarily by cells of the immune system that promote the proliferation and/or differentiative functions of other cells and play a significant rold in the regulation of the magnitude of the immune response. Examples of cytokines include interleukins, interferons, hematopoietic colony stimulating factors (CSF) and proinflammatory factors such as tumor necrosis factor (TNF). Such cytokines act on lymphocytes by stimulating them to proliferate thereby boosting a host's immune response. For example, IL-2 potentiates both innate or natural host defenses by stimulating NK cells and antigen-specific acquired immune reactivity by stimulating T cells and B cells.

Thus, the enhancement of an individual's immunity is a desirable goal in the treatment of patients diagnosed with cancer, autoimmune deficiency syndrome (AIDS), hepatitis and other disease states. Low dosages *e.g*., about 10-100 IU, of cytokines may be administered to patients in order to produce the desired immune response-enhancing ability of a cytokine in a host.

Additionally, the administration of lymphokines and related immunomodulators is a common therapy in many cancer treatments and has resulted in some positive responses in patients with various types of tumors. For example, high-dosages of interleukin 2 (IL-2) has demonstrated clinical activity in metastatic renal cell carcinoma and malignant melanoma with a small (5-15%) percentage of long term complete responses.

Interleukin-2 (IL-2), a cytokine which is primarily synthesized by activated T lymphocytes, plays a central role in the development of cell-mediated immunity. *See* Mertelsmann R. and Welte, K., Immunobiol. 172: 400-419 (1988). Low concentrations (*e.g*., 10-100 IU/ml) of IL-2 appear to be critical for the activation of antigen specific cytolytic lymphocytes as well as the clonal expansion of these effector cells. *See* Weiss, A., Fundamental Immunology, 3rd ed. New York: Raven Press, 467-504 (1993). Lotze et al. have shown that the exposure of murine or human lymphocytes to high concentrations of IL-2 (>600 IU/ml) over 3-4 days either *in vitro* or *in vivo* results in the proliferation of a population of cytotoxic lymphocytes termed lymphokine-activated killer (LAK) cells. *See* Lotze et al., Cancer Res., 41:4420-4425 (1981). Once activated, LAK cells demonstrate cytotoxicity against a wide variety of freshly isolated cancer cells or cultured cancer cell lines derived from syngeneic, allogeneic or even xenogeneic hosts. *See* Rayner et al., J. Natl. Cancer Inst., 75: 67-7; (1985); Hank et al., Cancer Res. 48: 1965-1971 (1988). Since the pattern of cytotoxicity is not tumor specific and does not require the expression of self-major histocompatibility complex (MHC) on target cells for cytotoxicity, LAK cells are termed "non-specific" killer cells. A major subset of the cytolytic cells in the LAK population are CD3 and express CD56, a neural cell adhesion molecule (N-CAM) thus indicating natural killer (NK) lymphocyte derivation. *See* Lanier et al., J. Immunol. 136: 4480-4486 (1986). Almost all freshly isolated and cultured malignant cells including multidrug-resistant tumor cells, are susceptible to LAK mediated cytolysis. *See* Harker et al., Cancer Res., 50: 5931-5936 (1990); Brune and Lapetina, Adv. Pharmaco/. 34: 351-360 (1995).

Further, IL-2 is an important lymphokine in the generation of anti-tumor immunity. In response to tumor antigens, a subset of lymphocytes, helper T cells, secrete a small amount of IL-2 which acts locally at the site of tumor antigen stimulation to activate cytotoxic T cells and natural killer cells that mediate systemic tumor cell destruction. It has been observed that the administration of IL-2 is able to induce the regression of certain cancers present in a mouse and in patients having metastatic cancers such as melanoma, cancer of the kidney, colorectal cancer or non-Hodgkin's lymphoma. *See* Rosenberg et al., N. Engl. J. Med. 316: 889-897 (1987). Further, clinical trials using IL-2 in combination with cytoreductive chemotherapy show promise in treatment of acute myelogenous leukemia, non-hodgkin's lymphoma and breast cancer.

Thus, the modulation of cytokine concentrations has been attempted as a means to enhance a patient's immune response. The administration of low dosages of cytokines such as IL-2 have been shown to stimulate or maintain a patient's immune response. *See* U.S. Patent No. 6,045,788. However, the clinical use of high dosages *e.g*., 100,000 to 600,000 IU, of cytokines such as IL-2, TNF-α, and interleukin-12 is limited by development of severe toxic side effects such as dose-dependent hypotension (systolic blood pressure (SBP) < 90 mm/Hg [< 11999.07 Pa]). The toxicity of systemically administered cytokines is not surprising as these agents mediate local cellular interactions and normally are secreted only in very small quantities. Unfortunately, the potentially lethal hypotensive side effect of therapeutic cytokine compositions severely limits the maximum dose of these compounds which can be given to a patient, thus reducing the immune activity and therapeutic anti-tumor effects of the cytokine.

Reactive oxygen species (ROS) and in particular, superoxide anions (O₂⁻) contribute to the unwanted side effects associated with cytokine therapy, such as hypotension, hyporeactivity and reduced T lymphocyte activation. Hypotension is a hemodynamic condition resulting from reduced vascular resistance despite increased levels of endogenous catecholamines. This condition persists despite the maintenance of normal blood volume (normovolemia). A characteristic of this condition is hyporeactivity, the loss of vascular responses, which develops to both exogenous and presumably, endogenous catecholamines.

Another characteristic of hypotension is the large increase in the production of free radicals, including superoxide anions within the body. *See* Ischiropoulos et al., Arch. Biochem. Biophys. 298: 446-451 (1992); Taylor et al., Arch. Biochem. Biophys., 316: 70-76 (1995). Administration of cytokines such as IL-2 releases superoxide anions which interact with and destroy the biological activity of vasopressor catecholamines, namely norepinephrine, epinephrine and dopamine. Superoxide anions are normally removed in biological systems by the formation of hydrogen peroxide and oxygen in the following reaction (hereinafter referred to as dismutation):

O₂⁻ + O₂⁻ + 2H⁺ - O₂ + H₂O₂

This reaction is catalyzed *in vivo* by the ubiquitous superoxide dismutase enzymes. Several non-proteinaceous catalysts which mimic this superoxide dismutating activity have been discovered. A particularly effective family of non-proteinaceous catalysts for the dismutation of superoxide consists of the manganese(II), manganese(III), iron(II) or iron(III) complexes of nitrogen-containing fifteen-membered macrocyclic ligands which catalyze the conversion of superoxide into oxygen and hydrogen peroxide, as described in U.S. Patents Nos. 5,874,421 and 5,637,578. *See also,* Weiss, R.H., et al., "Manganese(II)-Based Superoxide Dismutase Mimetics: Rational Drug Design of Artificial Enzymes", Drugs of the Future 21: 383-389 (1996); and Riley, D.P., et al., "Rational Design of Synthetic Enzymes and Their Potential Utility as Human Pharmaceuticals" (1997) in CatTech, I, 41. These mimics of superoxide dismutase have been shown to have a variety of therapeutic effects, including anti-inflammatory activity. *See* Weiss, R.H., et al., "Therapeutic Aspects of Manganese (II)-Based Superoxide Dismutase Mimics" in "Inorganic Chemistry in Medicine", (Farrell, N., Ed.), Royal Society of Chemistry, in Press; Weiss, R.H., et al., "Manganese-Based Superoxide Dismutase Mimics: Design, Discovery and Pharmacologic Efficacies" (1995), in "The Oxygen Paradox" (Davies, K.J.A., and Ursini, F., Eds.) pp. 641-651, CLEUP University Press, Padova, Italy; Weiss, R.H., et al., J. Biol. Chem., 271: 26149 (1996); and Hardy, M.M., et al., J. Biol. Chem. 269: 18535-18540 (1994). Other non-proteinaceous catalysts which have been shown to have superoxide dismutating activity are the salen-transition metal cation complexes, as described in U.S. Patent No. 5,696,109 and complexes of porphyrins with iron and manganese cations.

Currently, management of hypotension associated with cytokine administration includes infusion of fluids and albumin, and in some cases, treatment with catecholamine pressor agents such as dopamine. When dopamine fails to reverse hypotension, epinephrine, phenylephrine or noreprinephrine may be administered. However, despite repeated catecholamine doses, maintenance of an acceptable blood pressure (usually >90 mmHg [> 11999.02 Pa]) is often unattainable. Accordingly, in 20-50% of cases, intensive care unit (ICU) hospitalization for hemodynamic support is required as a result of unresponsive hypotension and hyporeactivity (loss of response to vasoconstrictors). As a result of the dose-limiting side effects, the full period of IL-2 administration is frequently curtailed in order to diminish hypotension and subsequent renal dysfunction.

Accordingly, a need presently exists for compositions for use in methods for enhancing the immune response resulting from the administration of a cytokine. Also needed are compositions for use in methods for preventing or treating hypotension which do not adversely affect the therapeutic mechanism of a cytokine. Further needed are compositions for use in methods for preventing and treating mammals suffering from hypotension resulting from cytokine administration by preventing the decrease of mean arterial pressure or by preventing and reversing the continued decrease of mean arterial pressure. Such compositions and methods would markedly facilitate IL-2 administration and would serve to broaden the clinical use of cytokines. Further needed are compositions for use in methods for enhancing the effectiveness of cytokine administration for cancer therapy by boosting the immune enhancing activity of the cytokine and/or by preventing and treating hypotension in mammals resulting from the administration of a cytokine.

### Summary of the Invention

According to the present invention there is provided a composition comprising interleukin-2, a pentaaza-macrocyclic ligand complex comprising a pentaaza-macrocyclic moiety chelated to a manganese (II) ion, and a pharmaceutically acceptable carrier, wherein the pentaaza-macrocyclic ligand complex has activity as a catalyst for the dismutation of superoxide and is selected from

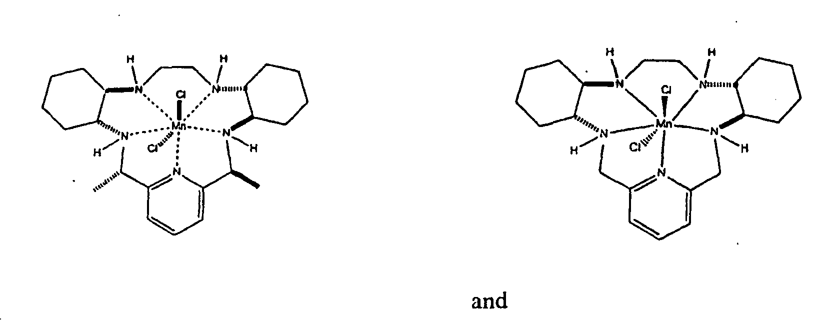

Without being limited to any particular theory, it is believed that the present invention enables the treatment of hypotension associated with the administration of a IL-2 by removing superoxide, thus protecting exogeneous and endogeneous catecholamines from autooxidation. By preventing or limiting the deactivation of catecholamines, hyporeactivity and hypotension are reversed and the effectiveness of IL-2 administration as a therapy for cancer can be improved.

According to the second aspect of the present invention there is provided A pentaaza-macrocyclic ligand complex selected from 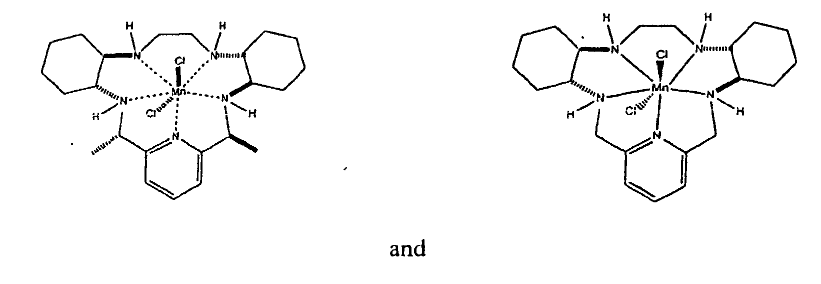 for use in inhibiting a fall in mean arterial pressure in a mammal due to the administration of interleukin-2, wherein a mean arterial pressure sustaining amount of the pentaaza-macrocyclic ligand complex is administered in conjunction with interleukin-2, and wherein the pentaaza-macrocyclic ligand complex has activity as a catalyst for the dismutation of superoxide.

According to the third aspect of the present invention there is provided a pentaaza-macrocyclic ligand complex selected from 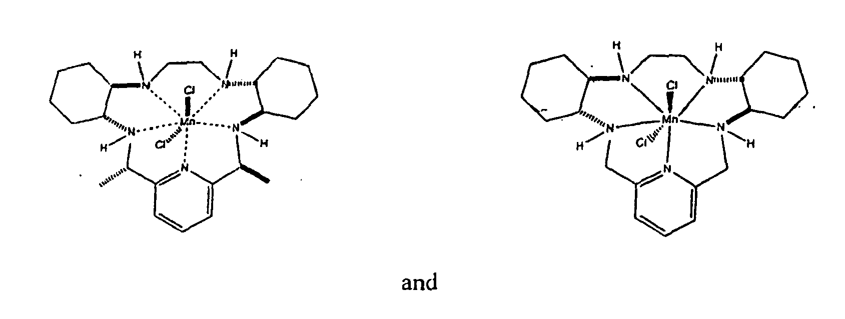 for the use in increasing mean arterial pressure in a mammal suffering from hypotension resulting from administration of interleukin-2, wherein a mean arterial pressure increasing amount of the pentaaza-macrocyclic ligand complex is administered in conjunction with interleukin-2, and wherein the pentaaza-macrocyclic ligand complex has activity as a catalyst for the dismutation of superoxide.

According to a fourth aspect of the present invention there is provided a pentaaza-macrocyclic ligand complex selected from 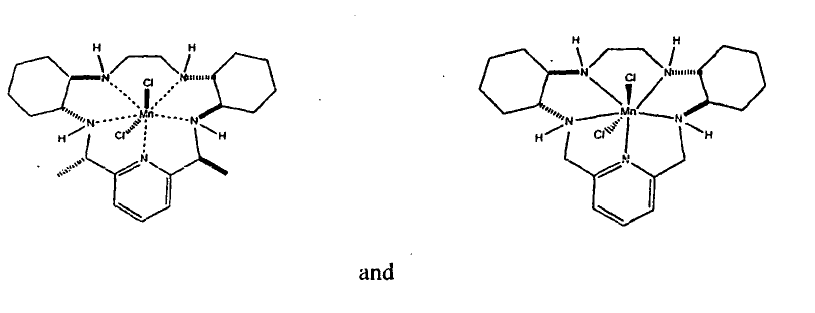 for use in a pharmaceutical composition for enhancing a mammal's immune response to cancer therapy, wherein a therapeutically effective amount of the pentaaza-macrocyclic ligand complex is administered in conjunction with interleukin-2 therapy, and wherein the pentaaza-macrocyclic ligand complex has activity as a catalyst for the dismutation of superoxide.

According to the fifth aspect of the present invention there is provided a pentaaza-macrocyclic ligand complex selected from 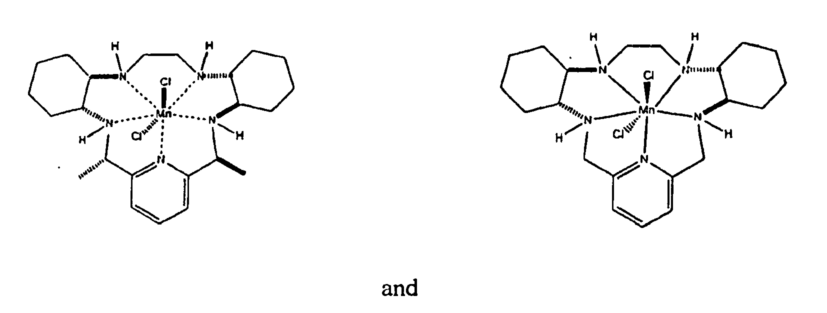 for use in a pharmaceutical composition for inhibiting the proliferation of a tumor in a mammal, wherein the composition comprising a therapeutically effective amount of the pentaaza-macrocyclic ligand complex and interleukin-2 administered to the mammal, and wherein the pentaaza-macrocyclic ligand complex has activity as a catalyst for the dismutation of superoxide.

Relatedly, pharmaceutical compositions are described which comprise a catalyst for the dismutation of superoxide alone or in combination with a IL-2 and a pharmaceutically acceptable carrier. Also discussed are pharmaceutical compositions which comprise a catalyst for the dismutation of superoxide, IL-2, a catecholamine pressor agent and a pharmaceutically acceptable carrier. When administered to a mammal afflicted with a tumor, these compositions enhance the immune response of the patient and the anti-tumor effect of cytokine therapy. Further, these pharmaceutical compositions inhibit the degradation of the catecholamines, allowing the catecholamine pressor agent to improve vascular tone and to increase the mean arterial blood pressure of the mammal. Other objects and features will be in part apparent and in part pointed out hereinafter.

### Brief Description of the Drawings

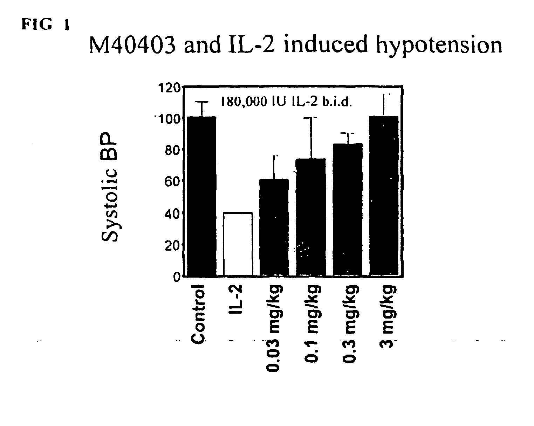

**Figure 1** is a graph indicating that IL-2 induced hypotension was attenuated in a dose-dependent manner by SOD mimetic M40403 with maximal reversal obtained at 3mg/kg M40403. At the highest dose of M40403 (3 mg/kg, twice a day (b.i.d.) for 5 days) there were no apparent signs of toxicity and particularly no effect on systolic blood pressure.

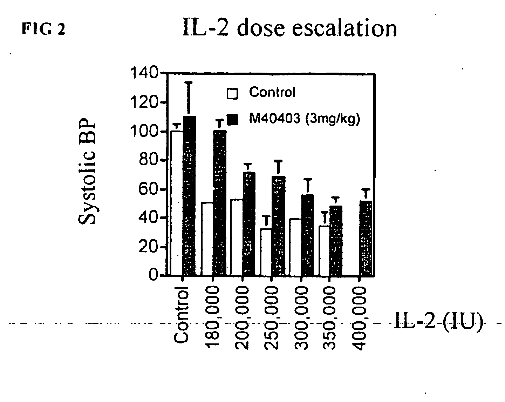

**Figure 2** is a graph indicating that higher doses of IL-2 could be administered (M40403 allowed BP to be maintained while IL-2 mediated death rates were reduced) in the presence of M40403. In the absence of M40403, there was 1 death in each IL-2 treated group at 200,000; 250,000 and 300,000 IU bid (1 out 6 died), all mice died in the 400,000 IU bid group (6 deaths of 6 mice). In the presence of M40403, all mice at all doses of IL-2 were alive (0 deaths of 6 mice in all groups).

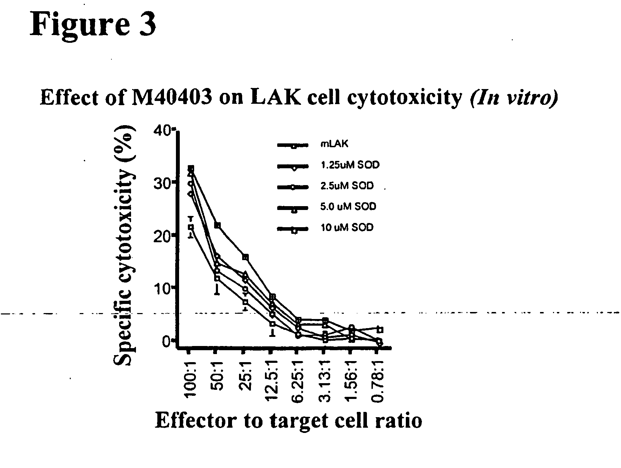

**Figure 3** is a graph indicating that a broad range of M40403 concentrations (0-10 µM) do not adversely affect murine (mouse) LAK cell activation.

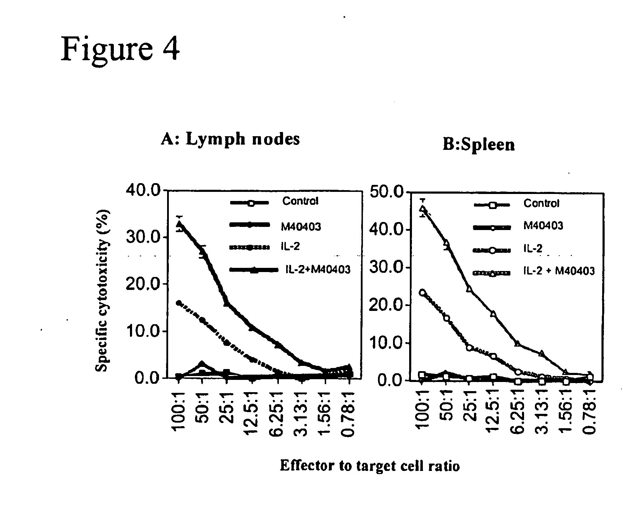

**Figure 4** is a graph which shows that SOD mimetic M40403 significantly potentiated IL-2 induced LAK cells activation *in vivo* as seen in the lymph nodes (A) and spleen (B). M40403, by itself did not increase LAK cell induction *in vivo.*

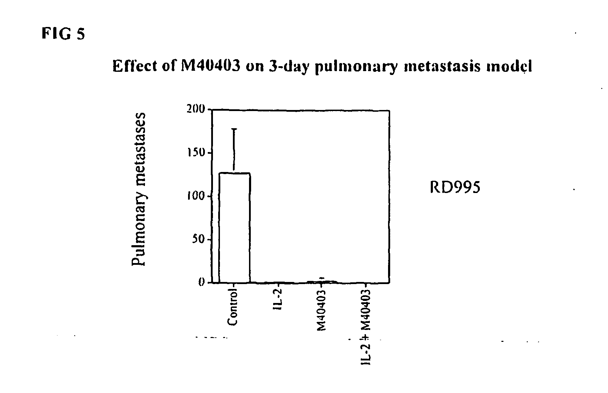

**Figure 5** is a graph which indicates that IL-2 treatment of mice eliminated the number of tumor nodules in lungs and does not affect the anti-tumor effects of IL-2. M40403 by itself at the dose used completely eliminated the numbers of tumor nodules in lungs of mice.

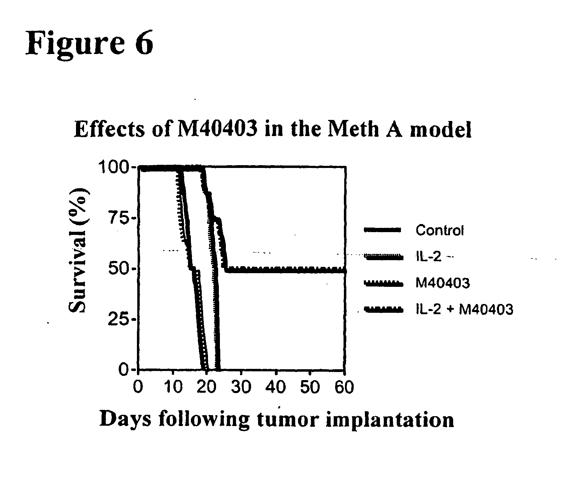

**Figure 6** is a graph indicating a survival advantage in IL-2 treated mice, compared to controls (14 days compared to 19 days). M40403 itself did not affect survival. These results suggest that M40403 not only blocks IL-2 induced hypotension but also improves IL-2 induced anti-tumor responses in a highly refractory treatment model.

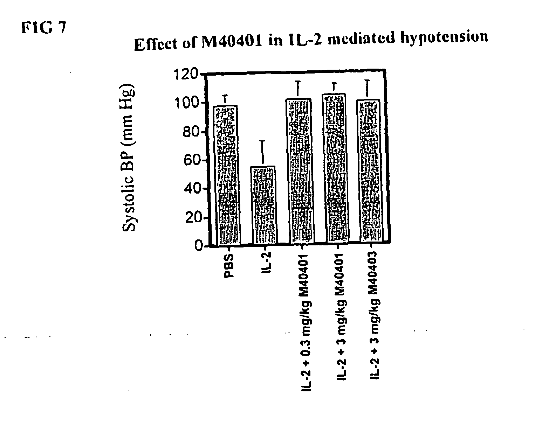

**Figure 7** is a graph which shows that M40401 did not interfere with the ability ofIL-2 to activate LAK cells *in vitro* and that M40401 reversed IL-2 mediated hypotension at lower doses than M40403. Full reversal of hypotension was seen at 0.3 mg/kg (b.i.d with IL-2 for 5 days, protocol identical to the one used for M40403).

In figures 1, 2 and 7 the units for systolic blood pressure are mmHg-to obtain the corresponding valves in Pa, the numbers indicated should be multiplied by 133, 32.

### Definitions and Abbreviations

To facilitate understanding of the invention, a number of terms as used herein are defined below:

The term "hypotension" means a hemodynamic condition characterized by lowered blood pressure which persists despite the maintenance of normal blood volume (normovolemia). Generally, hypotension occurs if the mean arterial pressure is less than 90 mmHg [11999.02 Pa] for at least one hour despite adequate ventricular filling pressures (pulmonary artery wedge pressure [PAWP] of 12 mmHg [1599.87 Pa]), or despite a sufficient central venous pressure (CVP) of 12 mmHg [1599.87 Pa]. Other indicators of hypotension are the failure of the hypotensive state to respond to aggressive initial fluid therapy (such as the administration of 500 ml of isotonic crystalloid, 25 gm or albumin, or 200 ml of other colloids (*e.g*. hydroxyethyl starch)), or the need for pressor doses of dopamine (> 5 g/kg/min), norepinephrine or other pressor agents to maintain a systolic blood pressure of 90 mmHg [11999.02 Pa.]

As used herein, "MAP" refers to mean arterial pressure.

The term "mean arterial pressure sustaining amount", as used in this application, means the amount of a compound needed to maintain the mean arterial pressure of a mammal suffering from or at imminent risk of suffering from hypotension associated with various shock states in the normotensive range, preferably from about 70 to about 130 mmHg [about 9332.57 to about 1733.91 Pa] for at least 30 minutes.

The term "mean arterial pressure increasing amount", as used in this application, means the amount of a compound needed to increase the mean arterial pressure of a mammal suffering from hypotension associated with various shock states from its hypotensive state to the normotensive range, preferably from about 70 to about 130 mmHg [about 9332.57 to about 17331.91 Pa] for at least 30 minutes. The phrase "co-administering a catalyst for the dismutation of superoxide in conjunction with interleukin-2" means that the catalyst is administered contemporaneous with, or sufficiently close in time before or after administration of the interleukin-2 so as to inhibit the hypotensive effects caused by the interleukin-2 administration and/or, as the context requires, sufficiently close in time to enhance interleukin-2-mediated therapeutic effects on the mammal's immune system and to inhibit the proliferation of a tumor.

The term "non-proteinaceous catalysts for the dismutation of superoxide" means a low-molecular weight catalyst for the conversion of superoxide anions into hydrogen peroxide and molecular oxygen. These catalysts commonly consist of an organic ligand and a chelated transition metal ion, preferably manganese(II), manganese(III), iron(II) or iron(III). The term may include catalysts containing short-chain polypeptides (under 15 amino acids), or macrocyclic structures derived from amino acids, as the organic ligand. The term explicitly excludes a superoxide dismutase enzyme obtained from any species.

The term "cancer therapy" refers to any treatment designed to kill tumor cells in a patient. As used herein, the terms "tumor cell" and "cancer cell" are used interchangeably to mean a malignant cell. A tumor cell can occur in and can be obtained from a solid tumor such as a sarcoma, carcinoma, melanoma, lymphoma or glioma or a more diffuse cancer such as a leukemia.

The mammal patient in the methods described herein is a mammal suffering from hypotension resulting from the administration of a cytokine or at imminent risk of hypotension due to the future administration of a cytokine. The term "mammal suffering from hypotension" is thus contemplated to include cases in which hypotension is anticipated, such as with imminent cytokine administration, as well as cases in which hypotension is apparent *e.g*., after cytokine administration. It is envisioned that a mammal patient to which the catalyst for the dismutation of superoxide will be administered described herein or in the compositions of the invention, will be a human. However, other mammal patients in veterinary (*e.g*., companion pets and large veterinary animals) and other conceivable contexts are also contemplated.

As used herein, the terms "treatment" or "treating" relate to any treatment of hypotension and include: (1) preventing hypotension from occurring in a subject; (2) inhibiting the fall of mean arterial pressure, *i.e*., arresting or limiting its development; or (3) ameliorating or relieving the symptoms of the disease. Additionally, as the context requires, the terms "treatment" or "treating" relate to any treatment of a subject for viral infection and include: (1) preventing viral infection from occurring in a subject; (2) inhibiting, arresting or limiting the development of the virus in the subject; or (3) ameliorating or reliving the symptoms of the viral infection.

### Detailed Description

This invention relates to compositions for use in methods for enhancing the immune-mediated activity of a interleukin-2 by administering therapeutic amounts of catalysts for the dismutation of superoxide alone or in combination with a low dosage of interleukin-2. Relatedly, the present invention is directed to compositions for us in materials for the prevention and treatment of hypotension associated with the administration of high dosages of interleukin-2 which comprises co-administering compositions containing a catalyst for the dismutation of superoxide in conjunction with interleukin-2. Preferred catalysts include superoxide dismutase enzyme (SOD) and small molecular weight organic ligand mimics of that enzyme (SOD mimetics or SODms).

Recent studies have indicated that macrophage synthesis of reactive oxygen species (ROS) such as superoxide ion (O₂⁻) may be a factor which inhibits the LAK cell activation process. Reactive oxygen species may be induced, in part as a result of inflammatory cytokines produced by LAK cells. The production of such inflammatory cytokines results in the upregulation of xanthine oxidase, an enzyme which generates superoxide as a byproduct. Thus, administration of cytokines such as IL-2 to a patient in need thereof releases superoxide ions which have been shown to interact and destroy the biological activity of vasopressor catecholamines *e.g*., norepinephrine, epinephrine and dopamine. Moreover, the inventor believe that superoxide (O₂⁻) reacts with catecholamines initiating a chain autooxidation reaction and deactivating them *in vitro.* Moreover, this deactivation may account for the hyporeactivity to exogenous catecholamines observed in cases of hypotension associated with cytokine administration. This suggests that the deactivation of endogenous norepinephrine by O₂⁻ contributes significantly to this aspect of the vascular crisis. The inventor has found that administration of a catalyst for the dismutation of superoxide protects these catecholamines from degradation and prevents both the hypotension and hyporeactivity associated with various shock states. *See* Macarthur et al., PNAS 97:9753 (2000).

Further, reactive oxygen species induce NFκB activation which may depress autocrine production of IL-2 and other cytokines that amplify LAK cell expansion. *See* Ginn et al., Free Radic. Biol. Med. 25: 346-361 (1998). Exposure of lymphocytes to oxidative radicals such as superoxide may result in lymphocyte anergy and apoptosis which in turn, limits the anti-tumor efficacy of IL-2. Accordingly, the removal of O₂⁻ will prevent and reduce the dose-limiting toxicity of hypotension resulting from IL-2 administration, thus allowing for continued administration of such cytokines. Further, the removal of superoxide will increase cytokine mediated LAK cell induction and potentiate the effectiveness of IL-2 as a cancer agent. The inventor believes that increased LAK cell activity may result in increased cytotoxicity by natural killer (NK) cells and perhaps T lymphocytes. An 8-18 fold increase in tumor cell killing (either via expansion of cytolytic cell numbers or via increased efficiency) could be translated into increased numbers of tumor cells killed and greater anti-cancer responses *in vivo*. Additionally, increased LAK cell activity may result in a possible shift towards increased TH1 helper cell response (pro-cytotoxic). The TH1 helper cell is a subset of helper T cells which may dictate the emergence of predominantly cytolytic immune responses versus predominantly antibody/DTH mediated responses based on cytokine secretion patterns. Induction of cytotoxicity is facilitated by IL-2, IFNγ, IL-12 (TH1 cytokine), and antibody responses appear to be induced by IL-4, IL-5, IL-6 with IL-10 acting to decrease TH1 activity (TH2 cytokine).

Accordingly, the present compositions include IL-2 and catalysts for the dismutation of superoxide to treat hypotension resulting from administration of IL-2. As a result, O₂⁻ is removed, hyporeactivity and hypotension associated with cytokine administration are reversed, and survival rate is improved. Depending on the condition of the mammal and the therapy used, the catalyst is administered before the administration of IL-2, administered contemporaneously with IL-2 or administered shortly after the administration of IL-2. Without being limited to a particular theory, it is believed that the administration of a composition containing IL-2 and a catalyst for the dismutation of superoxide enhances the cytokine immune response of the mammal to the tumor while preventing the potentially lethal hypotensive side effects associated with cytokine administration.

Relatedly, the invention believes that the administration of a composition containing a catalyst for the dismutation of superoxide in combination with a low dosage of IL-2 enhances the ability of IL-2 to stimulate an immune response thereby enhancing the cytokine immune response of the mammal to the tumor. In doing so, the ability of the host to inhibit the proliferation of a tumor is improved. As a result, IL-2 administration can be continued and the cancer treatment is enhanced.

### Catalysts for the dismutation of Superoxide

It is preferred that non-proteinaceous catalysts for the dismutation of superoxide be used in the compositions of the invention. The pentaaza-macrocyclic non-proteinaceous catalysts for use in the invention have catalytic activities which are close to or equal that of the enzymatic catalysts. Unlike the enzymes, the non-proteinaceous catalysts do not degrade in solution when stored for long periods of time at ambient temperatures and are considerably less antigenic. In addition, these catalysts are usually much simpler to synthesize and produce than enzymes, which must be isolated from natural sources or produced using recombinant biotechnology.

Non-proteinaceous catalysts for the dismutation of superoxide for use in the present invention preferably comprise an organic ligand and a transition metal cation. The catalysts are 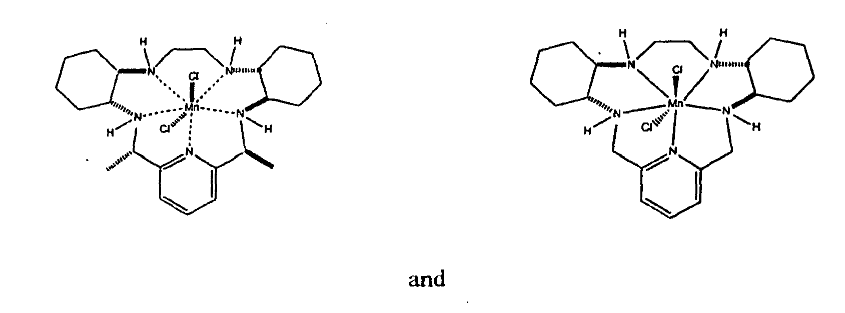 The pentaaza-macrocyclic ligand compound catalysts described have been further described in U.S. Patent No. 5,637,578, PCT application WO98/58636, and co-pending application U.S.S.N. 09/398,120, (now published as PCT application WO 01/19823). These pentaaza-macrocyclic ligand catalysts may be produced by the methods disclosed in U.S. Patent No. 5,610,293. However, it is preferred that the pentaaza-macrocyclic ligand compound catalysts used in the present invention be synthesized by the template method described in copending applications USSN 60/136,298 (now published as PCT application WO 00/72893) and USSN 09/398,120 (now published as PCT application WO 01/19823).

One compound for use in the present invention is designated compound M40401 and is represented by the following formula:

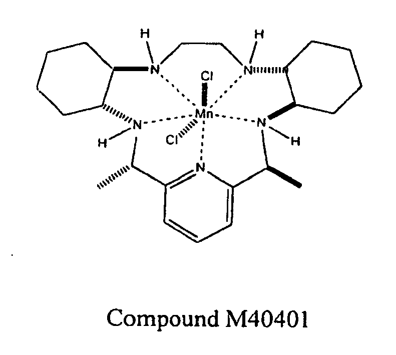

Another compound for use in the present invention is designated compound M40403 and is represented by the following formula:

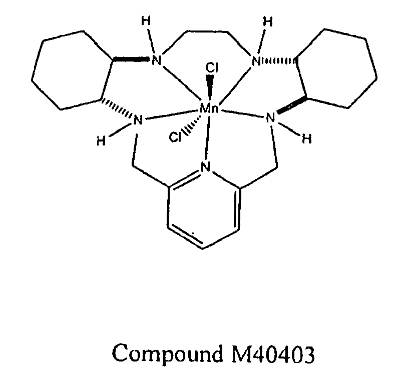

Activity of the compounds or complexes of the present invention for catalyzing the dismutation of superoxide can be demonstrated using the stopped-flow kinetic analysis technique as described in Riley, D.P. et al., Anal. Biochem., 196: 344-349 (1991). Stopped-flow kinetic analysis is an accurate and direct method for quantitatively monitoring the decay rates of superoxide in water. The stopped-flow kinetic analysis is suitable for screening compounds for SOD activity and activity of the compounds or complexes of the present invention, as shown by stopped-flow analysis, correlate to treating the above disease states and disorders.

In another preferred embodiment, the catalysts for the dismutation of superoxide are coupled with catecholamine pressor agents to be used in the compositions for use in methods for treating hypotension associated with the administration of a cytokine. Preferably, the catecholamine pressor agent is selected from dopamine, norepinephrine, epinephrine and alpha agonist phenyleprine, more preferably, dopamine and norepinephrine. Without being bound to any particular theory, the inventor proposes that the administration of a composition comprising a cytokine, a catalyst for dismutation of superoxide and a catecholamine pressor agent to a mammal suffering from hypotension resulting from cytokine administration will prevent the degradation of the catecholamines, thus allowing the catecholamine pressor agent to improve vascular tone and increase the mean arterial blood pressure of the mammal. Depending on the condition of the mammal and the therapy used, the catecholamine pressor agent is administered before the administration of the catalyst and cytokine or administrated contemporaneously with the catalyst and cytokine.

### Pharmaceutical Compositions

The compounds used in the invention can be formulated as pharmaceutical or veterinary compositions for use in the present invention. Depending on the subject to be treated, the mode of administration, and the type of treatment desired (*e.g*., inhibition, prevention, prophylaxis, therapy), the compounds are formulated in ways consonant with these parameters. The compositions of the present invention comprise a therapeutically or prophylactically effective dosage of a catalyst for the dismutation of superoxide alone or in combination with IL-2.

In a preferred embodiment, the pharmaceutical or veterinary compositions of the present invention comprise a therapeutically or prophylactically effective dosage of a catalyst for the dismutation of superoxide, administered concurrently with a IL-2. When administered to a mammal, these compositions enhance the ability of a IL-2 to stimulate an immune response thereby enhancing the IL-2 immune response of the mammal to the tumor. In doing so, the ability of the host to inhibit the proliferation of a tumor is improved.

In another preferred embodiment, pharmaceutical or veterinary compositions are provided which comprise the catalysts for the dismutation of superoxide, IL-2 and catecholamine pressor agents. When administered to a mammal in a state of hypotension resulting from the administration of a IL-2, these pharmaceutical compositions prevent or reduce the potentially lethal hypotension side effect associated with cytokine administration thereby allowing the continued administration of IL-2 to treat cancer. Preferably, these compositions prevent the degradation of catecholamines, allowing the catecholamine pressor agent to improve vascular tone and increase the mean arterial blood pressure of the mammal.

The compositions of the present invention may be incorporated in conventional pharmaceutical formulations (*e.g*. injectable solutions) for use in treating humans or animals in need thereof. Pharmaceutical compositions can be administered by subcutaneous, intravenous, or intramuscular injection, or as large volume parenteral solutions and the like. The term parenteral as used herein includes subcutaneous injections, intravenous, intramuscular, intrasternal injection, or infusion techniques.

For example, a parenteral therapeutic composition may comprise a sterile isotonic saline solution containing between 0.1 percent and 30 percent weight to volume of the catalysts for the dismutation of superoxide. A preferred solution contains from about 0.5 percent to about 10 percent, more preferably from about I percent to about 5 percent, and most preferably about 2.5 percent catalysts for dismutation of superoxide in solution (% weight per volume).

Injectable preparations, for example, sterile injectable aqueous or oleaginous suspensions may be formulated according to the known art using suitable dispersing or wetting agents and suspending agents. The sterile injectable preparation may also be a sterile injectable solution or suspension in a nontoxic parenterally acceptable diluent or solvent, for example, as a solution in ethanol and water. Among the acceptable vehicles and solvents that may be employed are water and isotonic sodium chloride solution. In addition, sterile, fixed oils are conventionally employed as a solvent or suspending medium.

Suppositories for rectal administration of the drug can be prepared by mixing the drug with a suitable nonirritating excipient such as cocoa butter and polyethylene glycols which are solid at room temperature but liquid at the rectal temperature and will therefore melt in the rectum and release the drug.

For administration to animal or human subjects, a typical dose of the composition comprising a catalyst for the dismutation of superoxide, catalyst for the dismutation of superoxide and a cytokine comprises 0.001 to about 10 mg of a catalyst for the dismutation of superoxide and a catecholamine pressor agent per kilogram of patient body weight and 100 to 600,000 I.U. of cytokine per kilogram of patient body weight. Preferably, the dosage of the catalyst for the dismutation of superoxide and catalyst for the dismutation of superoxide will range between 0.001 to 5 mg/kg patient body weight, more preferably 0.05 to 5 mg/kg body weight, and most preferably 0.05 to 1 mg/kg body weight. Preferably, the dosage of the cytokine in the composition will range between about 500 to about 100,000 I.U./kg body weight, more preferably about 1,000 to about 100,000 I.U./kg body weight and most preferably, about 5,000 to about 100,000 I.U./kg body weight. In another aspect of the invention, compositions may be administered to a patient to enhance the cytokine-mediated response against a virus. The typical dosages of such compositions comprising a catalyst for the dismutation of superoxide alone or in combination with a cytokine comprises 0.001 to about 10 mg of a catalyst for the dismutation of superoxide and preferably, 10-100 I.U. of cytokine per kilogram of patient body weight. For the above compositions, the total daily dose may be administered to a mammal in single or divided doses. Dosage unit compositions may contain such amounts of submultiples thereof to make up the total dose. However, one skilled in the art will recognize that the total dosage will vary on the particular composition being administered.

The amount of active ingredient that may be combined with the carrier materials to produce a single dosage form will vary depending upon the host treated and the particular mode of administration. It will be appreciated that the unit content of active ingredients contained in an individual dose of each dosage form need not in itself constitute an effective amount, as the necessary effective amount could be reached by administration of a number of individual doses. The selection of dosage depends upon the dosage form utilized, the condition being treated, and the particular purpose to be achieved according to the determination of those skilled in the art.

The dosage regimen for treating a disease condition with the compounds and/or compositions of this invention is selected in accordance with a variety of factors, including the type, age, weight, sex, diet and medical condition of the patient, the route of administration, pharmacological considerations such as the activity, efficacy, pharmacokinetic and toxicology profiles of the particular compound employed, whether a drug delivery system is utilized and whether the compound is administered as part of a drug combination. Thus, the dosage regimen actually employed may vary widely and therefore may deviate from the preferred dosage regimen set forth above.

The following examples are offered in order to illustrate but not to limit the present invention.

### EXAMPLES

### Example 1: Effect of SOD Mimic M40403 on IL-2 Induced Hypotension

IL-2, M40403 and IL-2/M40403 were administered to mice (n=6 per group) in order to determine the effect of these compounds on mean blood pressure. 180,000 IU of IL-2 was administered intraperitoneally (i.p.) twice daily for 5 days (10 doses) to each animal of the first group on a daily basis for 4 days. Experimental animals received M40403 (0.03-3 mg/kg) i.p.b.i.d. in conjunction with IL-2. Parallel groups of untreated mice or mice receiving M40403 alone (at the highest dose tested of 3 mg/kg) served as a control. Two hours after the last dose of IL-2, systolic blood pressure was measured via tail cuff (Stoelting, Wood Dell, IL) and using a PowerLab™ digital signal transducer (AD instruments, Mountain View, CA). Analysis was performed using Chart™ 3.6.1 software (AD instruments). The results of these tests can be found in Table 1.

**Table 1: Protective effects of M40403 on IL-2 induced hypotension**

| **Group** | **Mean Systolic BP** | **Standard Deviation** |
|---|---|---|
| Control | 82.6 | 3.47 |
| IL-2 | 44.53 | 7.85 |
| S.OD mimic | 111.21 | 45.31 |
| IL-2 + SOD mimic | 114.39 | 29.4 |

The blood pressure values represent the mean arterial blood pressure from the mean values of the six mice of each group. This experiment demonstrated striking (approximately 50%) reduction of systolic BP following IL-2 administration (mean 45±8.0 mmHg [5999.51 ± 1066.58 Pa]). Mice receiving the SOD mimic alone had variable increases in BP compared to controls (mean 111 ± 45 mmHg [14798.79 ± 5999.51 PA] versus 83 ± 4 mmHg [11065.76 ± 532.29 Pa] mostly error due to one very hypertensive mouse in SOD mimic group). Mice receiving the SOD mimic plus IL-2 had complete reversal of IL-2 induced hypotension (114 ± 29 mmHg [15198.75 ± 3866.35 Pa]).

As demonstrated in Figure 1, IL-2 induced hypotension was attenuated in a dose-dependent manner by M40403 with maximal reversal obtained at 3mg/kg M40403. At the highest dose of M40403 (3 mg/kg, b.i.d. for 5 days) there were no apparent signs of toxicity and particularly no effect on systolic blood pressure.

### Example 2: Dose escalation studies

The following studies were conducted to determine whether in addition to decreasing toxicity from IL-2, M40403 could be useful to increase the dose of IL-2 that can be tolerated by patients, or to assure that the full planned number of doses can be delivered, maximizing the potential for response.

Mice (n=6 per group) were treated with escalating doses of IL-2 of 180,000, 200,000, 250,000, 300,000, 350,000 and 400,000 IU IL-2 injected i.p. twice daily for 5 days (10 doses). Experimental animals received M40403 (3 mg/kg) i.p. b.i.d. in conjunction with IL-2. Two hours after the last dose of IL-2, systolic blood pressure was measured via tail cuff (Stoelting, Wood Dell, IL) and using a PowerLab™ digital signal transducer (AD instruments, Mountain View, CA). Analysis was performed using Chart™ 3.6.1 software (AD instruments).

In the absence of M40403, there was 1 death in each IL-2 treated group at 200,000, 250,000 and 300,000 I.U. b.i.d. (1 out 6 died), all mice died in the 400,000 I.U. b.i.d. group (6 deaths of 6 mice). In contrast, in the presence of M40403, all mice at all doses of IL-2 were alive (0 deaths of 6 mice in all groups). Accordingly, M40403 allowed BP to be maintained while IL-2 mediated death rates were reduced. Thus, higher doses of IL-2 may be safely administered in the presence of M40403.

### Example 3: Evaluation of the effect of M40403 on LAK cell induction in vitro

Studies were conducted to determine if M40403 affected the ability of IL-2 to induce LAK cells cytotoxicity *in vitro.* Murine splenocytes were incubated with increasing concentrations of M40403 during IL-2 activation (6000 IU/ml rhIL-2 in RPMI-1640 medium containing 10% fetal calf serum, antibiotics, and 1 mM glutamine). Non-M40403 exposed cultures served as a positive control. After 72h, LAK cells were harvested and cell viability evaluated. LAK cells cytotoxicity was tested against RD-995 tumor in triplicate samples at varying effector to target cell ratios (expressed as mean ±SD) using previously published methods (Yim et al., 1994; Samlowski et al., 1998).

These experiments established that a broad range of M40403 concentrations (0-10 µM) did not adversely affect murine (mouse) LAK cell activation (See Fig. 3). In fact, a dose-dependent increase of LAK cell activation appeared to be induced. Cell viability was assessed by trypan blue exclusion. Viability remained at 100% of control at up to 10µM M40403 (not shown). Similar results were obtained with M40403 when using human LAK cells (not shown).

### Example 4: Evaluation of the effect of M40403 on LAK cell induction in vivo

Mice (n=6 per group) were treated with 180,000 IU IL-2 injected i.p. twice daily for 5 days (10 doses). Experimental animals received M40403 (3 mg/kg) i.p. b.i.d. in conjunction with IL-2. Parallel groups of untreated mice or mice receiving M40403 alone (at the highest dose tested of 3 mg/kg) served as a control. Mice were sacrificed 2 hours following the last dose of IL-2. A single cell suspension was prepared from the lymph nodes and spleen from each animal, and serial dilutions made (starting from 10⁶). Cytolytic activity of lymph node and spleen cells was tested against ⁵¹Cr-radiolabeled RD-995 tumor (NK resistant, LAK sensitive, 10⁴ cells) in a standard 4h ⁵¹Cr release assay at a varying effector to target cell ratio (in triplicate). Parallel wells, containing 6000 IU/ml IL-2 were set up, to test for rapid upregulation of LAK activity which is found when IL-2 primed cytolytic effector cells are present. Statistical comparison of specific cytotoxicity (e.g. at 100:1 effector to target ratio) and lytic units per organ will be performed using Student's t-test, using Bonferroni's correction for multiple analyses.

As can be seen in Figure 4, M40403 significantly potentiated IL-2 induced LAK cells activation *in vivo* as seen in the lymph nodes (A) and spleen (B).

### Example 5: Enhancement of IL-2 induced tumor responses by M40403 in vivo.

The effects of M40403 on IL-2 induced tumor responses were assessed in two different tumors at an advanced stage of progression. RD-995 and Meth A have been chosen for initial experiments, because these tumors show differing sensitivity to IL-2 induced LAK cells, thus, allowing us to evaluate LAK mediated from non-LAK cell mediated tumor cell killing mechanisms induced by IL-2.

### Rosenberg tumor model.

RD-995 is a skin fibrosarcoma that was induced by chronic ultraviolet light exposure of a C3H/HeN mouse (Samlowski et al., 1995). This tumor grows in non-immunosuppressed mice, and is thus termed a "progressor" tumor. RD-995 is sensitive to LAK cell mediated killing *in vitro.* Pulmonary metastases derived from RD-995 are exquisitely sensitive to IL-2 treatment (with or without infusion of LAK cells). This model (known as the Rosenberg tumor model) was used in the following experiment to establish that M40403 does not diminish IL-2 induced responses in mice.

Four groups (n=6 mice per group) of mice (control, M40403 alone, IL-2 only, IL-2+M40403) were acclimated to metabolic cages. Once stabilized, mice were injected with 10⁶ RD-995 tumor cells intravenously. An IL-2 only treatment group served as a positive control, with IL-2 treatment beginning 3 days after tumor injection (IL-2 given at 180,000 IU i.p. every 12h for 5 days). Simultaneously, parallel groups of mice began M40403 (3 mg/kg), concurrently with IL-2. On day 14, mice were sacrificed by anesthetic overdose. After anterior thoracotomy, India ink solution (30 ml India ink, 4 drops ammonium hydroxide in 170 ml distilled water) was instilled into the trachea of each mouse via 18 ga. needle, until the lungs were completely expanded. Lungs were then excised and bleached in 5-10 ml Fekete's solution (100 ml 70% ethanol, 10 ml formaldehyde, 5 ml glacial acetic acid) for at least 24h prior to enumeration of metastases using a magnifying lens.

IL-2 treatment of mice eliminated the number of tumor nodules in lungs (Fig. 5). M40403 did not affect the anti-tumor effects of IL-2 (Fig. 5). However, M40403 by itself at the dose used completely eliminated the numbers of tumor nodules in lungs of mice (Fig. 5), an effect which was independent from LAK cell activation since it was found that M40403 by itself did not increase LAK cell induction *in vivo* (see Fig. 4). Based on the experimental data, Applicants believe that additive or synergistic effects of IL-2 plus M40403 are observed.

### Meth A fibrosarcoma tumor model.

Meth A was induced in a BALB/c mouse by methylcholanthrene treatment (obtained from Lloyd Old, MSKCC). This very aggressive fibrosarcoma is one of the rare tumors resistant to LAK cell mediated cytolysis *in vitro.* When injected intraperitoneally into mice (malignant ascites), this tumor responds to IL-2 therapy with improved survival. The mechanism is not completely established but may involve nitric oxide as a component of the treatment response. *See* Yim et al., J. Immunol. 155: 4382-4390 (1995). M40403, does not inhibit NO and does not affect NO-mediated apoptosis of Meth A tumor (not shown). The Meth A model serves to provide a resistant tumor model to evaluate potentially synergistic antitumor effects of combinations of IL-2 with M40403. This model is more representative of the majority of IL-2 treated human cancers, which at best undergo disease stabilization following IL-2 treatment followed by eventual disease progression.

Groups of 8 normal BALB/c mice (Control, M40403 only, IL-2 only, IL-2 + M40403) were injected with 2x10⁶ Meth A tumor cells intraperitoneally (day 0). On day 6 following tumor implantation, mice began treatment with IL-2 (180,000 IU s.c. every 12h for 5 days) with or without concomitant treatment with M40403 (3 mg/kg, s.c.). Survival of the mice was used as the major endpoint to assess therapeutic responses.

As shown in Figure 6, a modest but definite survival advantage occurs in IL-2 treated mice as compared to controls (14 versus 19 days). M40403 itself did not affect survival. In contrast, there was marked synergy between IL-2 and M40403, with 50% of mice still alive on day 60 with no visible (apparent) ascites. This result indicates that M40403 not only blocks IL-2 induced hypotension but also improves IL-2 induced anti-tumor responses in a highly refractory treatment model thereby enhancing the therapeutic effects of IL-2.

### Example 5: M40401 and IL-2

Preliminary experiments were also performed with M40401. These experiments demonstrated that M40401 does not interfere with the ability of IL-2 to activate LAK cells *in vitro.* Furthermore, and as expected from our previous data in septic shock, M40401 reversed IL-2 mediated hypotension at lower doses than M40403. Thus, full reversal of hypotension was seen at 0.3 mg/kg (b.i.d with IL-2 for 5 days, protocol identical to the one used for M40403) (See Fig. 7).

Other features, objects and advantages of the present invention will be apparent to those skilled in the art. The explanations and illustrations presented herein are intended to acquaint others skilled in the art with the invention, its principles, and its practical application. Those skilled in the art may adapt and apply the invention in its numerous forms, as may be best suited to the requirements of a particular use. Accordingly, the specific embodiments of the present invention as set forth are not intended as being exhaustive or limiting of the present invention.

## Claims

1. A composition comprising interleukin-2, a pentaaza-macrocyclic ligand complex comprising a pentaaza-macrocyclic moiety chelated to a manganese (II) ion, and a pharmaceutically acceptable carrier, wherein the pentaaza-macrocyclic ligand complex has activity as a catalyst for the dismutation of superoxide and is selected from

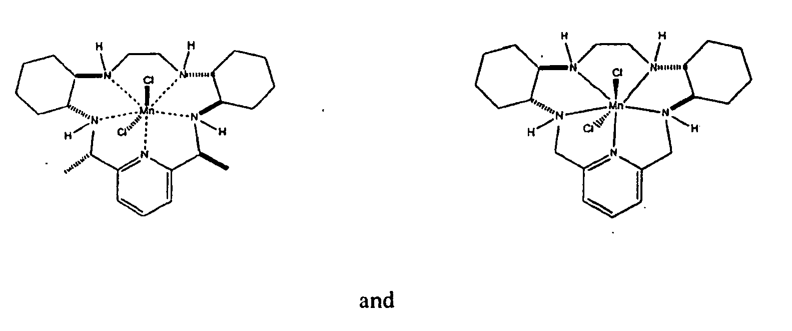

2. A composition according to Claim 1, wherein the composition further comprises a catecholamine pressor agent.

3. A composition according to Claim 2, wherein the catecholamine pressor agent is selected from dopamine, norepinephrine and epinephrine.

4. A pentaaza-macrocyclic ligand complex selected from 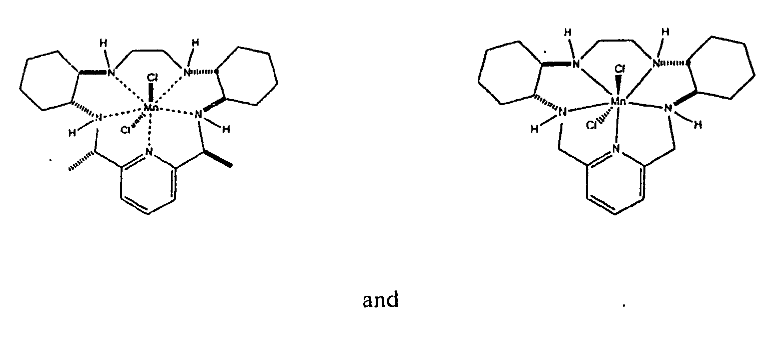 for use in inhibiting a fall in mean arterial pressure in a mammal due to the administration of interleukin-2, wherein a mean arterial pressure sustaining amount of the pentaaza-macrocyclic ligand complex is administered in conjunction with interleukin-2, and wherein the pentaaza-macrocyclic ligand complex has activity as a catalyst for the dismutation of superoxide.

5. A pentaaza-macrocyclic ligand complex selected from 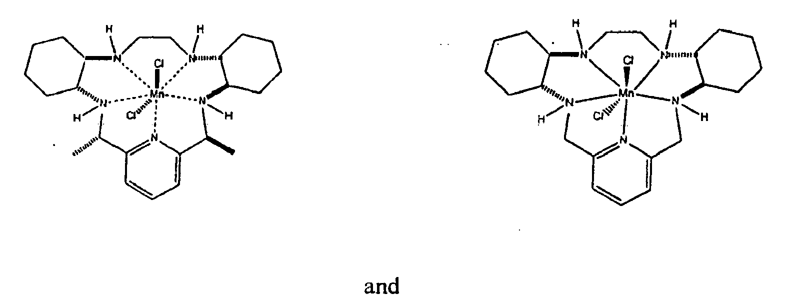 for the use in increasing mean arterial pressure in a mammal suffering from hypotension resulting from administration of interleukin-2, wherein a mean arterial pressure increasing amount of the pentaaza-macrocyclic ligand complex is administered in conjunction with interleukin-2, and wherein the pentaaza-macrocyclic ligand complex has activity as a catalyst for the dismutation of superoxide.

6. A pentaaza-marcocyclic ligand complex for use according to Claim 4 or Claim 5, wherein inhibition of the fall in mean arterial pressure or increase in mean arterial pressure is achieved by limiting autooxidation of catecholamines.

7. A pentaaza-macrocyclic ligand complex selected from 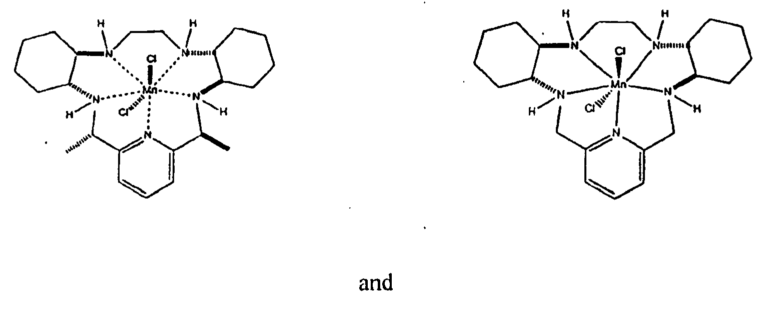 for use in a pharmaceutical composition for enhancing a mammal's immune response to cancer therapy, wherein a therapeutically effective amount of the pentaaza-macrocyclic ligand complex is administered in conjunction with interleukin-2 therapy, and wherein the pentaaza-macrocyclic ligand complex has activity as a catalyst for the dismutation of superoxide.

8. A pentaaza-macrocyclic ligand complex selected from 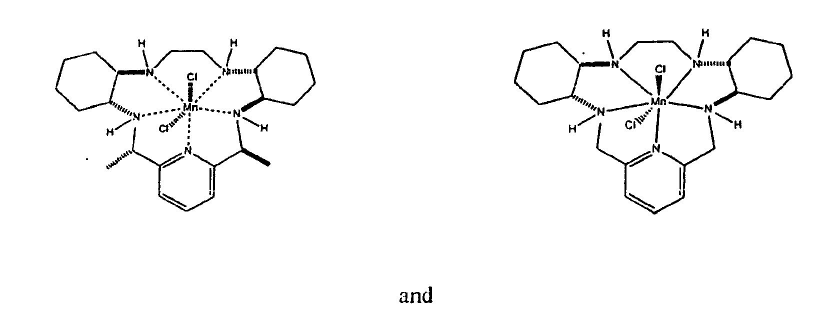 for use in a pharmaceutical composition for inhibiting the proliferation of a tumor in a mammal, wherein the composition comprising a therapeutically effective amount of the pentaaza-macrocyclic ligand complex and interleukin-2 administered to the mammal, and wherein the pentaaza-macrocyclic ligand complex has activity as a catalyst for the dismutation of superoxide.

9. A pentaaza-macrocyclic ligand complex for use according to any one of Claims 4 to 8, wherein the pentaaza-macrocyclic ligand complex is administered before or after the adiministration of the interleukin-2.

10. A pentaaza-macrocyclic ligand complex for use according to any one of Claims 4 to 8, wherein the pentaaza-macrocyclic ligand complex is administered contemporaneously with the interleukin-2.

11. A pentaaza-macrocyclic ligand complex for use according to any one of Claims 4 to 10, wherein the complex is co-administered with a catecholamine pressor agent.

12. A pentaaza-macrocyclic ligand complex for use according to Claim 11, wherein the catecholamine pressor agent is selected from dopamine, norepinephrine and epinephrine.

13. A pentaaza-macrocyclic ligand complex for use according to any one of Claims 4 to 12, wherein the administration may be carried out by intraarterial, intravenous, intramuscular or subcutaneous injection.

14. A pentaaza-macrocyclic ligand complex for use according to any one of Claims 4 to 3, wherein the mammal is a human.

15. A pentaaza-macrocyclic ligand complex for use according to any one of Claims 4 to 13, wherein the mammal is a companion pet.

16. A pentaaza-macrocyclic ligand complex for use according to any one of Claims 8 to 13, wherein the mammal is a large non-human animal.

## Patentansprüche

1. Zusammensetzung, die Interleukin-2, einen an ein Mangan(II)-Ion chelierten, einen pentaaza-makrocyclischen Teil aufweisenden, pentaaza-makrocyclischen Ligand komplex und einen pharmazeutisch zulässigen Träger aufweist, wobei der pentaaza-makrocyclische Ligandkomplex Aktivität als Katalysator für die Dismutation von Superoxid hat und unter 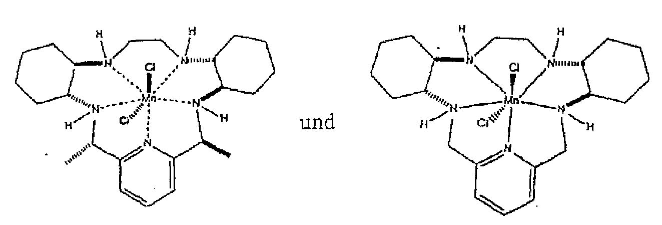 ausgewählt ist.

2. Zusammensetzung nach Anspruch 1, bei der die Zusammensetzung ferner ein Katecholamin-Blutdruckmittel enthält.

3. Zusammensetzung nach Anspruch 2, bei der das Katecholamin-Blutdruckmittel unter Dopamin, Norepinephrin und Epinephrin ausgewählt ist.

4. Pentaaza-makrocyclischer Ligandkomplex, der unter 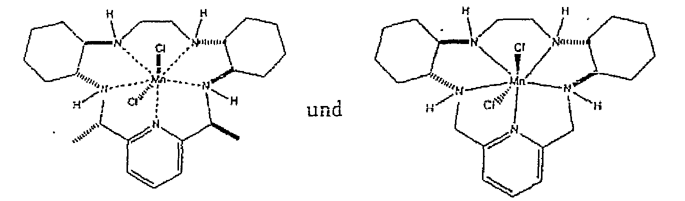 ausgewählt ist, zur Anwendung bei der Hemmung eines Abfalls des mittleren arteriellen Blutdrucks in einem Säuger infolge der Verabreichung von Interleukin-2, wobei eine den mittleren arteriellen Blutdruck aufrecht erhaltende Menge des pentaaza-makrocyclischen Ligandkomplexes zusammen mit Interleukin-2 verabreicht wird und wobei der pentaaza-makrocyclische Ligandkomplex eine Aktivität als Katalysator für die Dismutation von Superoxid hat.

5. Pentaaza-makrocyclischer Ligandkomplex, der unter 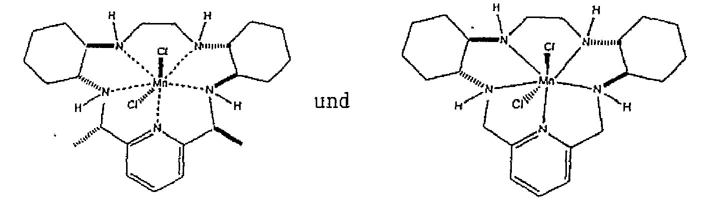 ausgewählt ist zur Anwendung bei der Erhöhung des mittleren arteriellen Blutdrucks in einem Säuger, der infolge von Verabreichung von Interleukin-2 unter niedrigem Blutdruck leidet, wobei eine den mittleren arteriellen Blutdruck erhöhende Menge des pentaaza-makrocyclischen Ligandkomplexes zusammen mit Interleukin-2 verabreicht wird und wobei der pentaaza-makrocyclische Ligandkomplex eine Aktivität als Katalysator für die Dismutation von Superoxid hat.

6. Pentaaza-makrocyclischer Ligandkomplex zur Anwendung nach Anspruch 4 oder Anspruch 5, bei dem die Hemmung des Abfalls des mittleren arteriellen Blutdrucks oder die Erhöhung des mittleren arteriellen Blutdrucks durch Begrenzung der Autoxidation von Katecholaminen erreicht wird.

7. Pentaaza-makrocyclischer Ligandkomplex, der unter 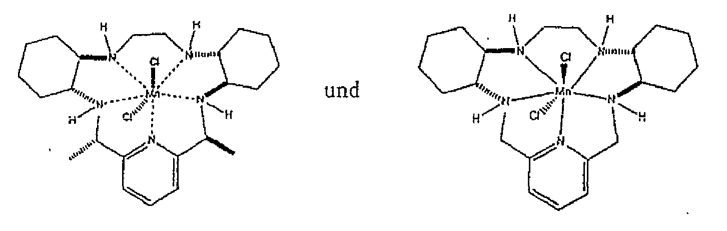 ausgewählt ist, zur Verwendung in einer pharmazeutischen Zusammensetzung zur Verstärkung der Immunreaktion eines Säugers auf Karzinombehandlung, wobei eine therapeutisch wirksame Menge des pentaaza-makrocyclischen Ligandkomplexes in Verbindung mit einer Interleukin-2-Therapie verabreicht wird und wobei der pentaaza-makrocyclische Ligandkomplex eine Aktivität als Katalysator für die Dismutation von Superoxid hat.

8. Pentaaza-makrocyclischer Ligandkomplex, der unter 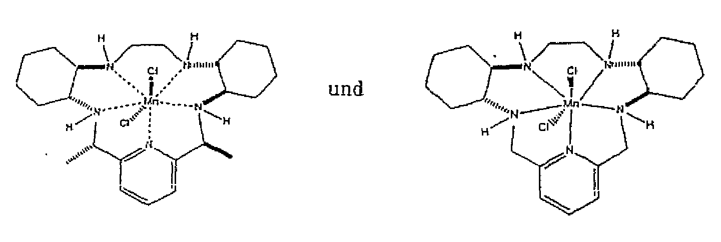 ausgewählt ist, zur Verwendung in einer pharmazeutischen Zusammensetzung zur Hemmung der Proliferation eines Tumors in einem Säuger, wobei die eine therapeutisch wirksame Menge des pentaaza-makrocyclischen Ligandkomplexes und Interleukin-2 enthaltende Zusammensetzung dem Säuger verabreicht wird und wobei der pentaaza-makrocyclische Ligandkomplex eine Aktivität als Katalysator für die Dismutation von Superoxid hat.

9. Pentaaza-makrocyclischer Ligandkomplex zur Anwendung nach einem der Ansprüche 4 bis 8, bei dem der pentaaza-makrocyclische Ligandkomplex vor oder nach der Verabreichung des Interleukins-2 verabreicht wird.

10. Pentaaza-makrocyclischer Ligandkomplex zur Anwendung nach einem der Ansprüche 4 bis 8, bei dem der pentaaza-makrocyclische Ligandkomplex gleichzeitig mit der Verabreichung des Interleukins-2 verabreicht wird.

11. Pentaaza-makrocyclischer Ligandkomplex zur Anwendung nach einem der Ansprüche 4 bis 10, bei dem der Komplex zusammen mit einem Katecholamin-Blutdruckmittel verabreicht wird.

12. Pentaaza-makrocyclischer Ligandkomplex zur Anwendung nach Anspruch 11, bei dem das Katecholamin-Blutdruckmittel unter Dopamin, Norepinephrin und Epinephrin ausgewählt ist.

13. Pentaaza-makrocyclischer Ligandkomplex zur Anwendung nach einem der Ansprüche 4 bis 12, bei dem die Verabreichung durch intraarterielle, intravenöse, intramuskuläre oder subkutane Injektion durchgeführt wird.

14. Pentaaza-makrocyclischer Ligandkomplex zur Anwendung nach einem der Ansprüche 4 bis 13, bei dem der Säuger ein Mensch ist.

15. Pentaaza-makrocyclischer Ligandkomplex zur Anwendung nach einem der Ansprüche 4 bis 13, bei dem der Säuger ein Haustier ist.

16. Pentaaza-makrocyclischer Ligandkomplex zur Anwendung nach einem der Ansprüche 8 bis 13, bei dem der Säuger ein großes nicht-menschliches Lebewesen ist.

## Revendications

1. Composition comprenant de l'interleukine-2, un complexe de ligand pentaaza-macrocyclique comprenant un fragment pentaaza-macrocyclique chélaté à un ion de manganèse (II), et un support pharmaceutiquement acceptable, dans laquelle le complexe de ligand pentaaza-macrocyclique possède une activité en tant que catalyseur pour la dismutation d'un superoxyde et est choisi parmi

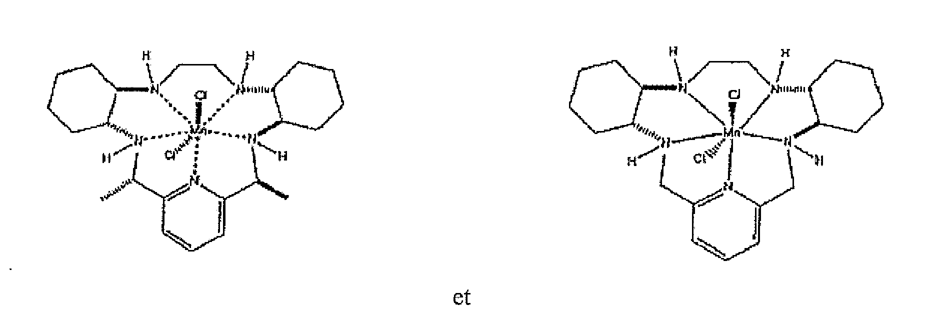

2. Composition selon la revendication 1, dans laquelle la composition comprend en outre un agent hypertenseur de type catécholamine.

3. Composition selon la revendication 2, dans laquelle l'agent hypertenseur de type catécholamine est choisi parmi la dopamine, la norépinéphrine et l'épinéphrine.

4. Complexe de ligand pentaaza-macrocyclique choisi parmi 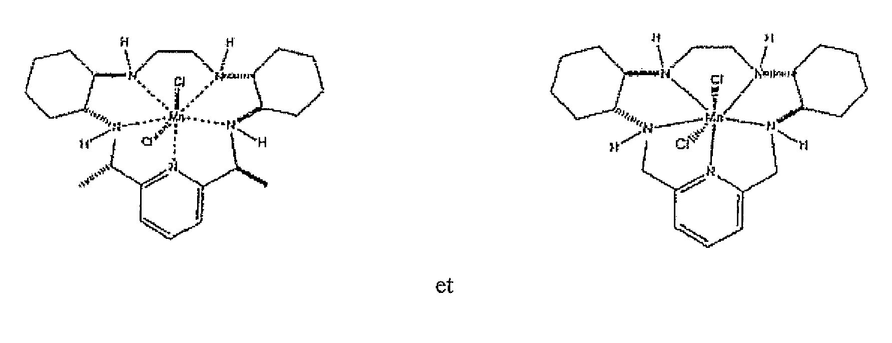 utilisable pour inhiber une chute de pression artérielle moyenne chez un mammifère due à l'administration d'interleukine-2, dans laquelle une quantité du complexe de ligand pentaaza-macrocyclique permettant de maintenir la pression artérielle moyenne est administrée en conjonction avec l'interleukine-2, et dans laquelle le complexe de ligand pentaaza-macrocyclique possède une activité en tant que catalyseur pour la dismutation d'un superoxyde.

5. Complexe de ligand pentaaza-macrocyclique choisi parmi 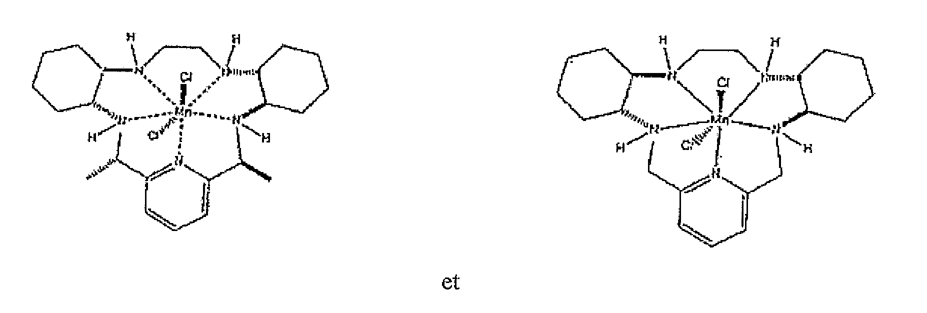 utilisable pour augmenter la pression artérielle moyenne d'un mammifère souffrant d'une hypotension résultant de l'administration d'interleukine-2, dans laquelle une quantité du complexe de ligand pentaaza-macrocyclique permettant d'augmenter la pression artérielle moyenne est administrée en conjonction avec l'interleukine-2, et dans laquelle le complexe de ligand pentaaza-macrocyclique possède une activité en tant que catalyseur pour la dismutation d'un superoxyde.

6. Complexe de ligand pentaaza-macrocyclique utilisable selon la revendication 4 ou la revendication 5, dans lequel l'inhibition de la chute de la pression artérielle moyenne ou l'augmentation de la pression artérielle moyenne est obtenue en limitant l'auto-oxydation des catécholamines.

7. Complexe de ligand pentaaza-macrocyclique choisi parmi 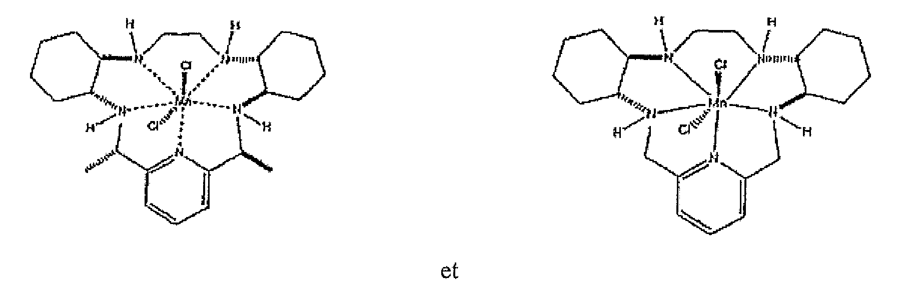 utilisable dans une composition pharmaceutique destinée à renforcer la réponse immunitaire d'un mammifère à un traitement anticancéreux, dans laquelle une quantité thérapeutiquement efficace du complexe de ligand pentaaza-macrocyclique est administrée avec un traitement par l'interleukine-2, et dans laquelle le complexe de ligand pentaaza-macrocyclique possède une activité en tant que catalyseur pour la dismutation d'un superoxyde.

8. Complexe de ligand pentaaza-macrocyclique choisi parmi 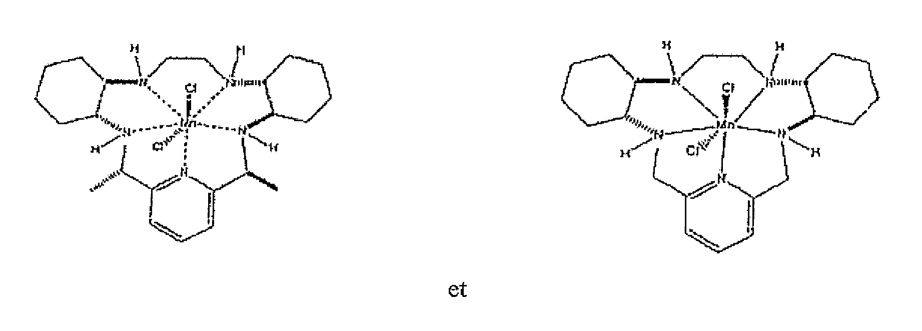 utilisable dans une composition pharmaceutique destinée à inhiber la prolifération d'une tumeur chez un mammifère, dans laquelle la composition comprend une quantité thérapeutiquement efficace du complexe de ligand pentaaza-macrocyclique et d'interleukine-2 administrée au mammifère, et dans laquelle le complexe de ligand pentaaza-macrocyclique possède une activité en tant que catalyseur pour la dismutation d'un superoxyde.

9. Complexe de ligand pentaaza-macrocyclique utilisable selon l'une quelconque des revendications 4 à 8, dans lequel le complexe de ligand pentaaza-macrocyclique est administré avant ou après l'administration de l'interleukine-2.

10. Complexe de ligand pentaaza-macrocyclique utilisable selon l'une quelconque des revendications 4 à 8, dans lequel le complexe de ligand pentaaza-macrocyclique est administré en même temps que l'interleukine-2.

11. Complexe de ligand pentaaza-macrocyclique utilisable selon l'une quelconque des revendications 4 à 10, dans lequel le complexe est co-administré avec un agent hypertenseur de type catécholamine.

12. Complexe de ligand pentaaza-macrocyclique utilisable selon la revendication 11, dans lequel l'agent hypertenseur de type catécholamine est choisi parmi la dopamine, la norépinéphrine et l'épinéphrine.

13. Complexe de ligand pentaaza-macrocyclique utilisable selon l'une quelconque des revendications 4 à 12, dans lequel l'administration peut être réalisée par injection intra-artérielle, intraveineuse, intramusculaire ou sous-cutanée.

14. Complexe de ligand pentaaza-macrocyclique utilisable selon l'une quelconque des revendications 4 à 13, dans lequel le mammifère est un humain.

15. Complexe de ligand pentaaza-macrocyclique utilisable selon l'une quelconque des revendications 4 à 13, dans lequel le mammifère est un animal de compagnie.

16. Complexe de ligand pentaaza-macrocyclique utilisable selon l'une quelconque des revendications 8 à 13, dans lequel le mammifère est un gros animal non humain.
